# EUROPEAN PATENT SPECIFICATION

(11) **EP 4 199 709 B1**
(45) Date of publication and mention of the grant of the patent: **05.11.2025**
(21) Application number: 21758265.9
(22) Date of filing: 30.07.2021
(51) Int. Cl.: A01K 29/00

(54) **SYSTEM AND METHOD FOR DETERMINING A HEALTH CONDITION OF AN ANIMAL**
SYSTEM UND VERFAHREN ZUR BESTIMMUNG DES GESUNDHEITSZUSTANDS EINES TIERES
SYSTÈME ET MÉTHODE DE DÉTERMINATION D'UN ÉTAT DE SANTÉ D'UN ANIMAL

(30) Priority: 23.09.2020 US 202063082241 P
(43) Date of publication of application: 28.06.2023
(73) Proprietor: Hill's Pet Nutrition, Inc., Topeka, KS 66603 (US)
(72) Inventor: WERNIMONT, Susan, Lawrence, Kansas 66049 (US); THOMPSON, Robin, Newcastle upon Tyne, Tyne and Wear NE2 3LJ (GB)
(74) Representative: Wichmann, Hendrik
(86) International application number: PCT/US2021/043833
(87) International publication number: WO 2022/066282

(56) References cited:
- JP-A- 2018 198 553
- US-A1- 2014 267 299
- US-A1- 2017 231 533
- US-A1- 2019 298 226
- US-A1- 2020 060 545
- US-A1- 2020 205 381

## Description

### BACKGROUND

An animal, such as a pet, is typically unable to communicate parameters relating to its health, such as the health status of the animal, the activity level of the animal, and diseases that the animal may be facing. Although the animal can be taken to a veterinarian for a checkup, such visits are often expensive and inconvenient, and the veterinarian may not have enough information to accurately diagnose the animal. Delayed treatment of the underlying issue may result in pain or even death to the animal. The movements of the animal may help in determining one or more conditions of the animal, such as the health status, activity level, and diseases that the animal may be facing.

Quantifying attributes of the movements of the animal, such as forward movements of the animal, may be useful for pet owners and veterinarians to evaluate the heath of the animal. One can manually observe the animal to determine how the animal moves in one or more directions. However, such manual approaches are often cumbersome and do not provide a timely diagnosis of the animal's health condition. Further, manual observation of animals is prone to inaccuracies, incompleteness, and forgetfulness. Thus, what is desired is a method and/or system for automatically determining the movements of the animal, for example, during a predetermined time period. Such determinations may be used to easily and accurately determining one or more conditions of the animal.
Different systems and methods for monitoring and/or displaying information related to health and wellness of an animal have been described. For example, US 2014/0267299 A1 describes a system and a method for displaying relevant health and wellness information to the veterinarian, wherein said information may come from the veterinarian's records as well as from a wearable device providing vital sign information. US 2017/0231533 A1 describes systems and methods for analyzing kinematics of an anatomical joint. JP 2018 198553 A, describes an activity management system which measures the activity state of an animal and is capable of calculating the residual activity amount required for the animal to reach an appropriate activity amount considering the type and age of the animal. US 2020/0060545 A1 describes a system and associated methods for round-the-clock monitoring of an animal's health status that includes an animal harness that is worn by the animal, and one or both of a mobile device and a remote server. US 2020/0205381 A1 describes a system, apparatus and method for determining a heath condition of an animal using data indicative of an event such as a discharge event and/or a feeding event of the animal. US 2019/0298226 A1 describes systems and methods for detecting lameness in a limb of an animal.

### BRIEF SUMMARY

The present invention relates to the methods and systems set out in the claims. A method of determining a health condition of an animal is provided. Movement data of the animal for a predetermined time period is received via a sensor (102, 302, 310, 410, 510a, 510b). The movement data includes at least one of an acceleration of the animal during the first predetermined time period, a velocity of the animal during the first predetermined time period, a distance traveled by the animal during the first predetermined time period, a location of the animal during the first predetermined time period, or steps taken by the animal during the first predetermined time period. A gait of the animal during the first predetermined time period is determined via one or more processors (110) based on the movement data of the animal during the first predetermined time period. A duration and/or a frequency of the gait of the animal during the first predetermined time period is determined. A time at which the animal is most active in the gait in the first predetermined time period is determined. A time at which the animal is least active in the gait in the first predetermined time period is determined. The time of the year of the first predetermined time period is recorded. Outdoor conditions at which the animal exercises the gait in the first predetermined time period are recorded. An activity level of the animal during the first predetermined time period is determined based on at least one of the duration or the frequency of the gait of the animal during the first predetermined time period. The activity level of the animal is caused to be displayed via a display device. Animal characteristic data comprising at least one of an age of the animal, a weight of the animal, a body type of the animal, a sex of the animal, a body fat index (BFI) of the animal, a body condition score (BCS) of the animal, or a muscle condition score (MCS) of the animal are received via one or more of the processors (110). The health condition of the animal is determined via the one or more processors (110), based on the animal characteristic data, the activity level, the gait of the animal during the first predetermined time period, the time of the year of the first predetermined time period, and the outdoor conditions at which the animal exercises the gait in the first predetermined time period.

A system for determining an activity level of an animal is provided. The system includes
a sensor (102, 302, 310, 410, 510a, 510b) configured to receive movement data of the animal for a first predetermined time period. The movement data includes at least one of an acceleration of the animal during the first predetermined time period, a velocity of the animal during the first predetermined time period, a distance traveled by the animal during the first predetermined time period, a location of the animal during the first predetermined time period, or steps taken by the animal during the first predetermined time period. The system further includes one or more processors (110) configured to determine a gait of the animal during the first predefined time period based on the movement data of the animal during the first predetermined time period; determine at least one of a duration or frequency of the gait of the animal during the first predetermined time period; determine a time at which the animal is most active in the gait in the first predetermined time period; determine a time at which the animal is least active in the gait in the first predetermined time period; record the time of year of the first predetermined time period; record outdoor conditions at which the animal exercises the gait in the first predetermined time period; determine an activity level of the animal during the first predetermined time period based on at least one of the duration or the frequency of the gait of the animal during the first predetermined time period; cause the activity level of the animal during the first predetermined time period to be displayed via a display device; receive animal characteristic data comprising at least one of an age of the animal, a weight of the animal, a body type of the animal, a sex of the animal, a body fat index (BFI) of the animal, a body condition score (BCS) of the animal, or a muscle condition score (MCS) of the animal; and determine a health condition of the animal based on the animal characteristic, the activity level, the gait of the animal during the first predetermined time period, the time of the year of the first predetermined time period, and the outdoor conditions at which the animal exercises the gait in the first predetermined time period.

### BRIEF DESCRIPTION OF THE DRAWINGS

The present invention will become more fully understood from the detailed description and the accompanying drawings, wherein:
FIG. 1 is a block diagram of a system having a plurality of modules configured to collect and analyze the behavior of an animal;
FIG. 2 is a perspective view of an example activity collar;
FIG. 3A is a depiction of an animal wearing the example activity collar of FIG. 2;
FIG. 3B is a depiction of another animal wearing the example activity collar of FIG. 2;
FIG. 4A is a perspective view of an example waste area having a sensor located on the waste area;
FIG. 4B is a perspective view of an example waste area with a sensor not located on the waste area;
FIG. 5A is a perspective view of an example feeding dish and drinking bowl having a sensor located on the feeding bowl and drinking bowl;
FIG. 5B is a perspective view of an example feeding dish and drinking bowl with a sensor not located on the feeding bowl or drinking bowl;
FIGS. 6A-6D are example screenshots of a use of the system of FIG. 1; and
FIG. 7 is an example use of the system, as described herein.

### DETAILED DESCRIPTION

The description of illustrative embodiments according to principles of the present invention is intended to be read in connection with the accompanying drawings, which are to be considered part of the entire written description. In the description of embodiments of the invention disclosed herein, any reference to direction or orientation is merely intended for convenience of description and is not intended in any way to limit the scope of the present invention. Relative terms such as "lower," "upper," "horizontal," "vertical," "above," "below," "up," 29 "down," "top," and "bottom" as well as derivatives thereof (e.g., "horizontally," "downwardly," "upwardly," etc.) should be construed to refer to the orientation as then described or as shown in the drawing under discussion. These relative terms are for convenience of description only and do not require that the apparatus be constructed or operated in a particular orientation unless explicitly indicated as such. Terms such as "attached," "affixed," "connected," "coupled," "interconnected," and similar refer to a relationship wherein structures are secured or attached to one another either directly or indirectly through intervening structures, as well as both movable or rigid attachments or relationships, unless expressly described otherwise. Moreover, the features and benefits of the invention are illustrated by reference to the exemplified embodiments. The scope of the invention is defined by the claims appended hereto.

As used throughout, ranges are used as shorthand for describing each and every value that is within the range. Any value within the range can be selected as the terminus of the range.

The system and method of the invention determine an activity level of an animal based on movement data of the animal which data are used to determine a gait during a predetermined time period, as defined in the claims. The movement may be a forward movement, such as the forward gait of the animal. The monitoring of the animal according to the method or system of the invention is used to determine an activity level of the animal or a health condition of the animal. Example animals may include a pet (*e.g.,* cat, dog, bunny, guinea pig, bird), a farm animal (*e.g.,* a horse, cow, chicken), a zoo animal (*e.g.,* a lion, bear), an animal in the wild, and the like. As described herein, the monitoring of the animal's motion provides (*e.g.,* automatically provides) an indication (*e.g.,* general indication) of the animal's health condition. Specifically, the health condition of the animal is determined based on the animal characteristic data, the activity level, the gait of the animal during the first predetermined time period, the time of the year of the first predetermined time period, and the outdoor conditions at which the animal exercises the gait in the first predetermined time period as defined in the claims. The monitoring of the animal may provide a detection (*e.g*., an early detection) of an animal's health abnormality, such as sickness, disease, injury, lameness, obesity, arthritis, *etc.* The detection may be possible because the health abnormality (*e.g*., injury) may result in the gait of the animal changing. Example injuries may include strains, sprains, bone fractures, joint dislocations, *etc.* For example, injuries may include cranial cruciate ligament ruptures and/or patellar luxations, which may result in lameness in dogs. Example injuries may include common injuries (*e.g*., a twisting of the ankle) and/or traumatic injuries (*e.g*., from an auto accident). The health abnormality may include dementia. For example, an animal experiencing dementia may walk during the night and sleep during the day. The health monitoring of the animal may provide *(e.g.,* automatically provide) an indication of the activity of the animal, such as tracking/evaluating the activity of the animal per day, checking/evaluating whether a boarded pet was exercised, *etc.*

The health monitoring of the animal may provide (*e.g*., automatically provide) a marker for training of the animal, a marker for various life stages/states of the animal, such as aging or obesity of the animal. The health monitoring of the animal may provide a tool to estimate energy usage of the animal and/or may be used as a metric for disease states, such as joint issues such as arthritis, hip dysplasia, lameness, foot injury, osteopathic disease, weakness and/or deconditioning (*e.g*., due to age), neurological disorders, and the like. The monitoring of the animal's movements may result in many benefits to the animal, especially if the animal's caretaker or the animal's doctor takes corrective action as result of a detected health abnormality. For example, the system and/or method may be designed for use at the home of the animal and may lead to vital information being provided to the animal's care taker and/or animal doctors.

The system for determining an activity level of an animal according to the invention may include one or more devices and/or mechanisms worn by an animal for receiving, determining, storing, and/or transmitting information of the animal, such as the sensor (102, 302, 310, 410, 510a, 510b) which is comprised by the system of the invention. The mechanisms may be worn on one or more of the head of the animal, the ears of the animal, the neck of the animal, the torso of the animal, limbs (*e.g.*, arms, legs) of the animal, the tail of the animal, the mouth (*e.g.,* tooth, cap over the tooth, replacement tooth), the eye (*e.g.,* contact lenses), and the like. The mechanisms may be placed in one or implants within the animal, such as implants within the belly and/or base of the tail of the animal, a neuticle of the animal, *etc.* The devices may be coupled to a collar, harness, bracelet, anklet, belt, earring, headband, and the like. In other examples, the devices may be attached to one or more attachment mechanisms, such as a coat, boot, decorative clothing (*e.g*., ribbon), sweater, hat, *etc.* In other examples one or more of the devices and/or mechanisms may be implanted within the animal. For example, one or more of the devices and/or mechanisms may be a subdermal implant that may be placed underneath the skin of the animal.

A recognition device *(e.g.,* coupled to the mechanism worn by the animal) may identify the animal within the system. The animal may be linked to an animal profile. The animal's movements may be monitored, tracked, and/or electronically recorded *(e.g.,* automatically monitored, tracked, and/or electronically recorded) on a predefined frequency *(e.g.,* on a daily, weekly, monthly, yearly basis). The animal's movement data are received via a sensor (102, 302, 310, 410, 510a, 510b) and used to determine a gait of the animal during a first predetermined time period, and the gait is used in determining the animal's health condition and activity level, as defined in the claims herein. The animal's movement may be monitored, tracked, and/or recorded without disturbing the animal or disrupting its natural behavior.

The monitoring of the animal's movement may be performed via a collection of one or more types of data. The data may include motion data, location data, orientation data, spatial data, and the like. The data may be collected and/or monitored during one or more of the animal's activities, such as walking, trotting, cantering, ambling, pacing, galloping, and the like. The data may be collected and/or monitored to determine the pace of the animal. The data may relate to the animal moving in one or more directions, such as in a forward direction, reverse direction, sideward direction, vertical direction, *etc.* Collected data may be stored in a repository that may be accessible to animal caregivers, veterinarians, and the like. The data may be accessible via a portable electronic device (*e.g.,* an application of a portable electronic device) and/or a server.

A portable electronic device may be one or more of a number of devices, including without limitation, a smart phone, a cell phone, a tablet computer, a personal digital assistant ("PDA"), a laptop computer, *etc.* The data may be analyzed to identity behavior and/or habits of the animal, and to provide the data and/or advice to owners based on the data. The data may be collected and/or generated over time, for example, for statistical processing of the animal's movements. The data may be compared with previously collected and/or stored data for purposes of understanding the animal's health trends, variations in an animal's state of health, for determining the activity level of the animal, for determining whether a health abnormality exists for the animal, *etc.* The previously collected and/or stored data may relate to the animal that is being monitored and/or the previously collected and/or stored data may relate to another animal (*e.g.,* for comparison purposes).

An animal's health condition, such as whether the animal is suffering from an injury/illness/disease, and/or the activity of the animal, is determined by the method of the invention based on the animal characteristic data, the activity level, the gait of the animal during a first predetermined time period (which is determined based on the movement of the animal), the time of the year of the first predetermined time period, and the outdoor conditions at which the animal exercises the gait in the first predetermined time period, as defined in the claims. The health condition of the animal may be recorded. To determine the health condition of the animal by the method of the invention, parameters indicative of the animal's health condition are monitored and/or recorded including movement data received via a sensor (102, 302, 310, 410, 510a, 510b) and animal characteristic data comprising at least one of an age of the animal, a weight of the animal, a body type of the animal, a sex of the animal, a body fat index (BFI) of the animal, a body condition score (BCS) of the animal, or a muscle condition score (MCS) of the animal. For instance, the amount of times the animal walks, runs, jogs, gallops, rests, *etc.* in a time period, the duration of the walking, trotting, cantering, ambling, pacing, galloping, resting, *etc* is recorded. As an example, the time period of the running of the animal may be an hour per day, a minute per day, and the like.

Application of statistical methods may be used to derive information about the animal's health condition based on the movement of the animal. For example, a healthy animal may be expected to run for a minimum and/or maximum amount of time during a time period (*e.g.,* per day, per week, per month, *etc.*) A mean and median of the above parameters may be defined for a healthy animal and/or for an unhealthy animal. If the animal performs a defined health parameter (*e.g*., runs) for less than, or more than, an amount defined for a healthy animal, the animal may be identified as being unhealthy (*e.g*., a sick, injured, diseased, *etc*.). In other examples if the animal performs a defined health parameter (*e.g.,* runs) for less than, or more than, an amount defined for a healthy animal, the animal may be identified as not being exercised and/or trained as often as desired. As defined in the claims, animal characteristic data comprising at least one of an age of the animal, a body type of the animal, a sex of the animal, The body fat index (BFI), body condition score (BCS), muscle condition score (MCS), and/or weight of the animal are received via one or more processors (110) and used to determine, via the one or more processors (110), the health condition of the animal.

Application of statistical methods may be used to derive information about an animal's health condition based on the movement of a single animal, the movement of more than one animal, movement(s) of similar animals, movement(s) of different animals, or a combination thereto. The derived information may be used to form a metric, matrix, and/or an index, such as a health metric, a health matric, and/or a health index. One or more characteristics of the animal's movement (such as evenness of weight distribution among the animal's legs, distance between foot strikes of the animal, right/left sided differences of the animal, right/left sided speed of the animal, and/or a combination of foot strikes of the animal used to form the gait of the animal *(e.g.,* two beats, three beats, four beats, *etc*.)) may be used to form a health metric or health index. The health metric or health index may be a characteristic of a life stage *(e.g.,* young vs. old) of the animal, a disease condition *(e.g.,* arthritis) of the animal, or the like.

Characteristics of the animal are used used to determine whether an animal's movements are indicative of a healthy animal or an unhealthy animal. Said animal characteristics include at least one of an age of the animal, a weight of the animal, a body type of the animal, a sex of the animal, a body fat index (BFI) of the animal, a body condition score (BCS) of the animal, or a muscle condition score (MCS) of the animal, and may further include the species, breed, geographic location, stage of life, size, *etc.* of the animal. For example, a dog may be expected to move faster and/or farther than a cat. As a result, a running distance per day that is desired for a cat may not be a sufficient running distance for a dog. In another example, a running distance per day that is desired for a dog may not be a sufficient distance for a horse. Parameters determined, identified, received, and/or transmitted may be recorded. The parameters may be recorded continuously, for example, from the moment of system activation throughout animal's life. In other examples, the parameters may be recorded for a predefined time period (*e.g.,* for a day, a week, a month, *etc*.), on a predefined frequency (*e.g.,* every weekday), *etc.*

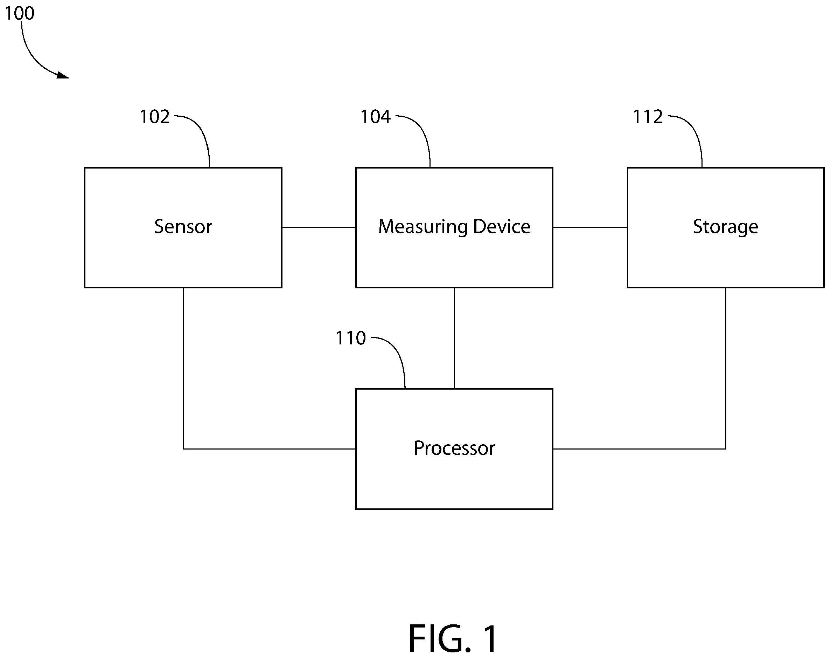

FIG. 1 shows an example system for monitoring an animal's behavior, health, habits, and/or other characteristics. System 100 may include a sensor 102, a measuring device 104, and/or a storage device 112.

Sensor 102 may be configured to detect a location of the animal, to detect the motion (or stillness) of the animal, to detect an orientation of the animal, *etc.* Sensor 102 may be one or more of a variety of form factors, including, but not limited to, an accelerometer, a gyroscope, a magnetometer, force transducers, displacement transducers, pressure transducers, force sensors, displacement sensors, pressure sensors, load cells, photographic cameras, video cameras, camcorders, audio sensors, and a combination thereof. In examples, sensor 102 may include one or more of thermometers, electrocardiography (ECG), photo plethysmography (PPG) devices, microphones, respiratory inductive plethysmography (RIP) devices, optoelectronic plethysmography (OEP) devices, or transthoracic impedance devices. For example, caloric expenditure may be assessed by heat produced, by cardiac/respiratory output, distance traveled, and/or step metrics. ECG and PPG may provide pulse/heart rate detection. Microphones, RIP, OEP and impedance may provide a breathing rate.

In addition, or alternatively, sensor 102 may be one or more of optical sensors, optical reflecting sensors, LED/photodiode pair optical sensors, LED/phototransistor pair optical sensors, laser diode/photodiode pair optical sensors, laser diode/phototransistor pair optical sensors, optocouplers, optical fiber coupled optical sensors, magnetic sensors, ultrasonic sensors, microphones, weight sensors, force sensors, displacement sensors, pressure sensors, various proximity sensors, such as inductive proximity sensors, magnetic proximity sensors, capacitive proximity sensors, and/or a combination thereof. Sensor 102 may include communication circuitry, such as Bluetooth (*e.g.*, classic Bluetooth and/or Low Energy Bluetooth), RFID, Wi-Fi, and other wireless technologies. Sensor 102 may communicate with one or more devices, for example, sensor 102 may communicate with a server.

Measuring device 104 may be configured to measure a characteristic related to the animal. Measurement device 104 may be a device that is separate from sensor 102 or a device that is the same as sensor 102. Example measuring devices 104 may be implemented in one or more of a variety of form factors, including, but not limited to, weighing scales, weight transducers, force transducers, displacement transducers, pressure transducers, weight sensors, force sensors, displacement sensors, pressure sensors, real time clocks, timers, counters, and/or a combination thereof. Measuring device 104 may include communication circuitry, such as Bluetooth (*e.g*., classic Bluetooth and/or Low Energy Bluetooth), RFID, Wi-Fi, Medical Implant Communication System (MICS) *(e.g.,* a hybrid of the technologies, such as MICS/Bluetooth), and other wireless technologies, and other wireless technologies. Measuring device 104 may communicate with one or more devices, for example, measuring device 104 may communicate with a server.

Storage device 112 may be configured to store data provided to and/or from system 100. The data may include motion data and/or location data provided by the sensor 102, for example. Example storage devices 112 may be memory devices, data storage devices, and a combination thereof, such as memory chips, semiconductor memories, Integrated Circuits (IC's), non-volatile memories or storage device such as flash memories, Read Only Memories (ROM's), Erasable Read Only Memories (EROM's), Electrically Erasable Read Only Memories (EEROM's), Erasable Programmable Read Only Memories (EPROM's), Electrically Erasable Programmable Read Only Memories (EEPROM's), an Electrically Erasable Programmable Read Only Memory (EEPRO), volatile memories such as Random Access Memories (RAM's), Static Random Access Memories (SRAM's), Dynamic Random Access Memories (DRAM's), Single Data Rate memories (SDR's), Dual Data Rata memories (DDR's), Quad Data Rate memories (QDR's), microprocessor registers, microcontroller registers, CPU registers, controller registers, magnetic storage devices such as magnetic disks, magnetic hard disks, magnetic tapes, optical memory devices such as optical disks, compact disks (CD's), Digital Versatile Disks (DVD's), Blu-ray Disks, Magneto Optical Disks (MO Disks) and/or a combination thereof. In one embodiment, the storage device comprises a semiconductor RAM IC for an intermediate recording of the behavior, health, and/or characteristics of the animal, and then transfer of the data to a flash memory IC for non-volatile recording. Storage 112 may be an external memory device, such as a USB flash memory, an external hard drive, *etc.*

System 100 may include a processor 110 configured to calculate and/or process data provided to system 100, for example. Example processors may be electronic circuits, systems, modules, subsystems, sub modules, devices, and combinations thereof, such as Central Processing Units (CPU's), microprocessors, microcontrollers, processing units, control units, tangible media for recording and/or a combination thereof. Storage device 112 may be configured to store derived data from the processor 110. Processor 110 may include communication circuitry, such as Bluetooth (*e.g.*, classic Bluetooth and/or Low Energy Bluetooth), RFID, Wi-Fi, and other wireless technologies. Processor 110 may communicate with one or more devices, for example, processor 110 may communicate with a server.

In an example, sensor 102, and/or storage 112 may be assembled in a number of configurations, including in a stand-alone apparatus. In another example, sensor 102, storage 112, and processor 110 may be assembled in a stand-alone apparatus. In other examples, the processor 110 and/or storage 112 may be configured as remote devices, such as remote servers (e.g., cloud storage devices). Although FIG. 1 shows a connection between processor 110 and each of sensor 102, measuring device 104, and storage 112, examples should not be so limited. In examples one or more of the devices may communicate with one or more (including any, or none) of the other devices. For example, sensor 102 may communicate with processor 110 and storage 112, sensor 102 may not communicate with storage 112, *etc.* One or more devices may be added and/or removed from system 100. For example, additional sensors 102 may be added to system 100 and/or storage 112 may be removed from system 100.

Data relating to the movement(s) of the animal is processed and/or recorded for a determination of the animal's activity level and/or health condition as defined in the claims. For example, the amount of times, durations, *etc.,* that an animal walks, trots, canters, ambles, paces, gallops, and/or rests may be used to determine a health condition of an animal, an activity level of the animal, *etc.* A weight of an animal, a body temperature of an animal, the date and/or time of an event (*e.g.,* a walking, trotting of the animal), the time of an event (*e.g.,* a walking, trotting, cantering), and/or the time of a movement of the animal may be used to determine a health condition of an animal. One or more activities of the animal may be recorded via a video recording, picture, and/or audio recording and/or may be processed.

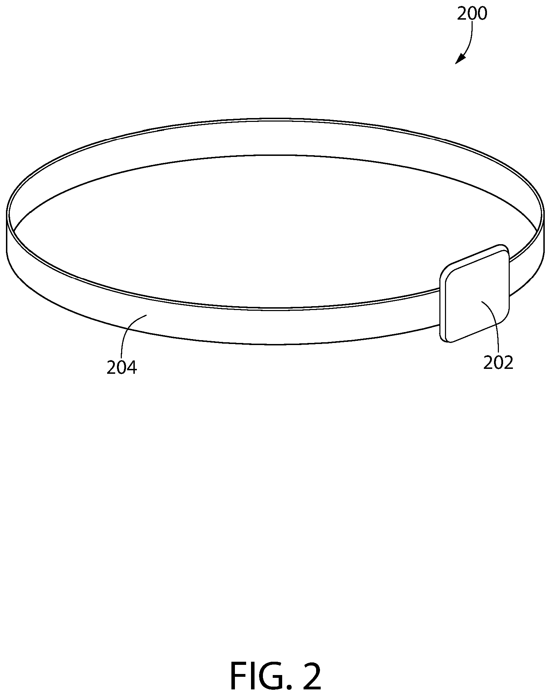

FIG. 2 is a perspective view of an example mechanisms 200 worn by an animal. Although FIG. 2 shows mechanism 200 as a collar, it should be understood that mechanism 200 may be one or more mechanisms worn by an animal and/or constraining the animal. For example, collar, mechanism 200 may include a collar, harness, bracelet, anklet, belt, earring, headband, and the like. In other examples devices that may house or couple to an electronic device may include one or more attachment mechanisms, such as a coat, boot, decorative clothing (*e.g.,* ribbon), sweater, hat, *etc.* Mechanism 200 may be used to constrain the animal, store information about the animal, and/or transmit information relating to the animal.

Mechanism 200 may be linked to a particular animal (*e.g.*, may be linked to a profile of a particular animal). Mechanism 200 may include circuitry 202 that may include a processor, storage, wireless communication hardware, one or more sensors (*e.g*., accelerometers, gyroscopes, magnetometers, *etc*.), location device (*e.g.,* proximity beacon, GPS, satellite-based location systems, Bluetooth based positioning/tracking systems, cellular based location systems, and the like), temperature sensors, moisture detectors, biometric sensors, *etc.* The location devices (*e.g.,* a cellular based location system) may be used to triangulate the location of the animal. In examples the location device may measure a distance (*e.g*., relative distance) to another device (such as a user's mobile) device. By determining the relative distance to another device (which may have one or more of an accelerometer, gyroscope or cellular service), the absolute distance of the animal may be determined. The wireless communication hardware may include a transmitter and a receiver. For example, the wireless communication hardware of the mechanism 200 may include a low energy communication device, such as Bluetooth Low Energy or RFID. Mechanism 200 may include a Medical Implant Communication System (MICS), Bluetooth, or a hybrid of the technologies, such as MICS/Bluetooth). The mechanism 200 may include a memory for storing data. Circuitry 202 may be coupled to a collar of mechanism 200, such as collar 204.

An accelerometer located on the mechanism 200 may be configured to measure motions of the animal. For example, the accelerometer may measure accelerations of the animal, changes in velocity of the animal, and/or changes in position of the animal. A gyroscope may be configured to measure changes in orientation of the animal and/or changes in rotational velocity of the animal. A magnetometer may be configured to measure orientation (*e.g.*, absolute orientation) of the animal, for example, in the NESW plane.

As described above, the mechanism 200 may include a location device, such as a proximity beacon, a GPS, *etc.* The location device may track a position of the animal. For example, the location device may indicate that the animal is inside a home, outside a home, *etc.* For example, the location device may indicate that the animal is within a park (*e.g.,* a dog park), within an exercise area (such as an exercise area of a dog boarding kennel), within a crated area, *etc.* The movement of the animal may be associated with the location of the animal. For example, the acceleration/velocity/speed and/or distance that an animal travels may be greater when the animal is outside a home versus when the animal is located inside a home. As a result, an animal may be expected to run more when located outside a home than when located inside a home. For example, an animal located outside that does not run beyond a predefined distance and/or for a predefined time (*e.g.*, based on the body type, breed, sex, *etc.,* of the animal) may be determined to have an abnormal condition, whereas an animal located inside that does not run beyond a predefined distance and/or for a predefined time (*e.g.,* based on the body type, breed, sex, *etc.,* of the animal) may not be determined to have an abnormal condition. The movement of the animal is associated with a time of year and outdoor conditions in which the animal is located. For example, dogs may run less in cold temperatures (*e.g.,* in January) or rainy weather than in mild temperatures (*e.g.,* in April) or sunny days. Dogs may scratch more on high pollen count days than lower pollen count days.

The location of the animal (such as being located within home) for periods of time may be used to determine whether the animal is provided the conditions to achieve the desired amounts of exercise. Such information may be used to determine whether the caregiver of the animal should provide additional or less outside time and/or exercise routines for the animal. When the animal is located indoors for an amount of time less than a predefined amount of time, or when the animal moves for a distance and/or time that is less than a predefined amount of distance and/or time, automated alerts may be sent to the system so that the caregiver, pet parent, veterinarian, and/the like can be notified. Based on these alerts, the caregiver of the animal may adjust the amount of time in which the animal is located outdoors, as well as adjust the exercise (*e.g*., time and/or distances of exercise) obtained by the animal. The location of the animal and the movements of the animal may be correlated. For example, the movement of the animal while the animal is being boarded may be determined. Such information may be useful to determine the level that the animal was exercised while being boarded.

Mechanism 200 may send data relating to an animal to a server, electronic device (*e.g*., mobile phone of pet parent or caregiver), and the like. For example, mechanism 200 may send motion data (including gait data), orientation data, location data, *etc.,* to a server, electronic device, *etc.* The server may perform computations of the data, for example, to determine whether the amount and/or duration of the movement of the animal is desired. The server may determine a signature of the animal based on the movement of the animal. The signature of the animal may be compared with signatures of abnormal (*e.g*., diseased, obese, *etc*.) animals and/or signatures of normal (*e.g.,* non-diseased) animals. The server may be configured to communicate the data to the user and/or to one or more other parties (*e.g.,* a veterinarian, pet parents, care givers, *etc*.). In examples, an electronic device (*e.g.,* the care giver's mobile phone) may perform computations of the data to determine whether the movement of the animal is above, or below, predefined levels desired for the animal. The electronic device may be configured to communicate the data to the user and/or one or more other parties (*e.g.,* a veterinarian, spouse, *etc*.).

The mechanism 200 may have a biometric monitoring sensor. The biometric monitoring sensor may be configured to determine body measurements and/or calculations of the animal. For example, temperature sensor and/or heart rate sensor may be used to determine the body temperature of the animal and/or the heart rate of the animal. The biometric monitoring sensor may be located on the activity collar or on another device position on or about the animal.

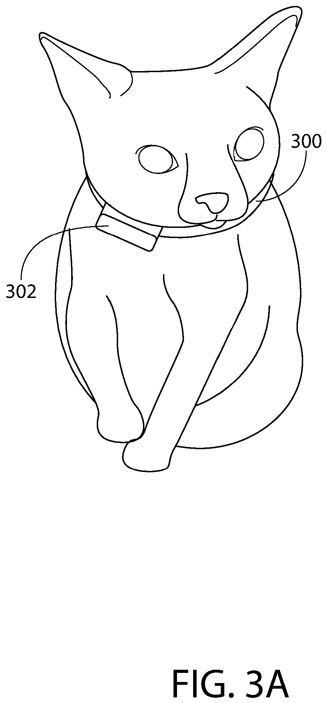

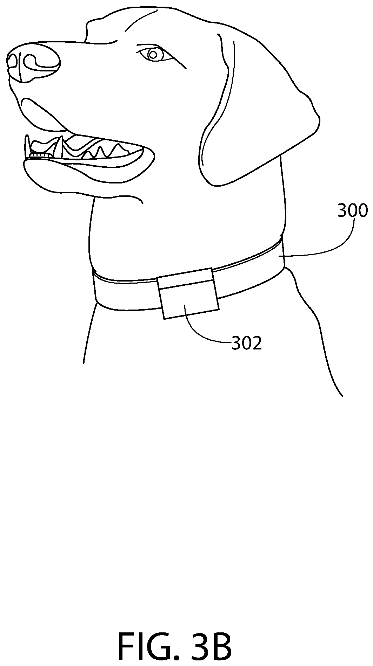

FIGS. 3A, 3B show example uses of the mechanism 300. As shown on FIG. 3A, a cat may wear the mechanism 300, such as in the form factor of an activity collar. As shown on FIG. 3B, a dog may wear the mechanism 300. Although the examples shown on FIGS. 3A, 3B are collars, it should be understood that the collar (*e.g.*, activity collar) is for illustration purposes only and mechanism 300 may be any device (*e.g*., wearable device) that may come in other form factors besides a collar, as described herein. For example, mechanism 300 may be a jacket, vest, hat, gloves, contact lenses, rings *(e.g*., earrings), or any other device (or combinations of devices) that can be worn on the outside (or inside) of an animal. In other examples mechanism 300 may be any device and/or area in which the animal may be proximate, such as a waste area, a feeding area, a play area, *etc.,* as described herein.

As described herein, the mechanism 300 (*e.g.,* activity collar) may have one or more sensors 302, such as an accelerometer. The sensor 302 may be coupled to the mechanism 300, for example, on an outside of the mechanism 300. In other examples, the sensor (*e.g.*, accelerometer) may be integrally formed within the mechanism 300. As shown on FIG. 3A, a location sensor 310 may be included in the system. The location sensor 310 may be located on the animal (*e.g.*, worn by the animal) or positioned upon a surface that is not the animal. The location sensor 310 may be a proximity sensor. For example, a proximity sensor may be used to determine if the animal is near a predefined area, such as a feeding bowl, water bowl, and/or waste area.

According to the invention the sensor (102, 302, 410, 510a, 510b) is used to receive movement data of the animal for a first predetermined time period. Said movement data comprise at least one of an acceleration of the animal during the first predetermined time period, a velocity of the animal during the first predetermined time period, a distance travelled by the animal during the first predetermined time period, a location of the animal during the first predetermined time period, or steps taken by the animal during the first predetermined time period, and may further ocmprisethe direction, duration, *etc.* in which the animal is moving. Movement of the animal may be determined based on motion data, orientation data, location data, *etc.,* of the animal. The sensors and other devices may be used to determine the location at which an animal is moving. The location at which the animal is moving may be useful for determining whether the animal is healthy or unhealthy, such as whether the animal is running in play areas or hiding in rest areas. The location at which the animal is moving may be useful for determining whether the animal is exhibiting desired behaviors or undesired behaviors. The location at which the animal is performing the animal event may be useful for determining whether the animal is performing behaviors at desired or undesired locations. For example, an animal may be expected to be resting while indoors (such as in the bed of the animal) and/or the animal may be expected to be moving at a predefined velocity (or speed) and for a predefined duration when the animal is outdoors (such as at the dog park).

As provided herein, the activity collar may provide motion data, orientation data, *etc.,* of the animal. In addition, a location data of the animal may be provided, for example, via a proximity sensor. The motion data, orientation data, and/or location data may be provided via devices worn by, or not worn by, the animal. For example, location data may be provided via a device proximate to the animal, such as a proximity sensor that may be located on a feeding area and/or waste area.

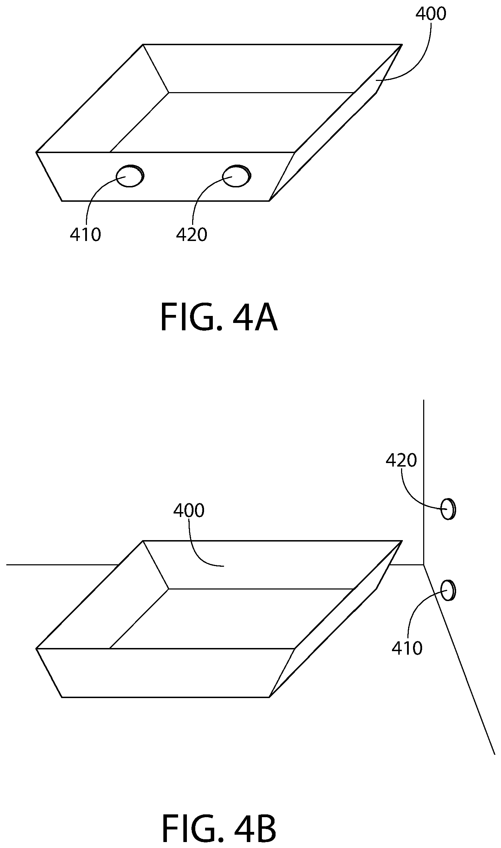

FIGS. 4A, 4B show example waste areas that may be used to monitor an animal's movements, for example, to determine a gait of the animal and/or other activities of the animal (such as defecating, urinating, vomiting, *etc*.). A waste area may be any area that an animal uses for emptying its bowels, bladder, and a combination thereof regularly, periodically, or occasionally. FIG. 4A shows an example waste area 400 (waste area 400) that includes one or more devices, such as a proximity sensor and/or measuring device. The proximity sensor may be a camera, scale, a motion detector, an RFID tag, an RFID reader, a proximity beacon, a GPS, a passive infrared, a microwave, an ultrasound, *etc.* The proximity sensor may be used to track the movement of the animal. For example, the proximity sensor may be used to track the acceleration, velocity, speed, duration, frequency, direction, *etc.,* of the animal's movement (*e.g.*, gait) over a predefined time.

The animal's behaviors and/or habits relating to the animal's gait may be monitored at the waste area 400 using the sensors, devices (*e.g.,* measuring devices), *etc.,* located at or on the waste area. The animal's gait may also, or alternatively, be monitored at the waste area using the sensors, devices (*e.g.,* measuring devices), *etc.,* located on an animal (*e.g.,* an activity collar), as described herein. The litter box 400 may track the distance and/or acceleration/velocity/speed in which the animal approaches the litter box 400, exits the litter box, moves proximate the litter box, *etc.* Although waste area 400 is shown as a litter box, it should be understood that a waste area may be form factors other than a litter box. For example, a waste area may be a designated area (*e.g*., inside a house or outside) in which an animal may defecate, urinate, and/or vomit. The designed area may include a backyard, a papered area, a toilet, a cage (such as a birdcage, *etc*.).

As shown on FIG. 4A, waste area 400 may be an area designated for an animal (*e.g.,* a cat) to urinate and/or defecate. Waste area 400 may have one or more sensors. The one or more sensors may be example sensor 410, shown on FIGS. 4A and 4B. As shown on FIG. 4A, sensor 410 may be located on a portion of the waste area, such as waste area 400. Sensor 410 may not be located on a portion of the waste area. For example, as shown on FIG. 4B, sensor 410 may be located on a wall, a table, *etc*., or any other surface that may be in a predefined proximity to the waste area. Sensor 410 may be a motion sensor (such as an accelerometer, a gyroscope, a magnetometer, *etc*.), a proximity sensor, an orientation sensor, a location sensor, and/or one or more other sensors, as described herein. Waste area 400 may include communication circuitry, such as Bluetooth, RFID, Wi-Fi, and other wireless technologies. Waste area 400 may communicate with an activity collar (such as mechanism 300) and/or a server. Waste area 400 may communicate directly with a portable electronic device of the user, or such communication may occur indirectly via a server and an application, such as a web application.

As described herein, waste area 400 may include a proximity sensor, such as a camera, scale, *etc.,* to track the movements, such as the directions, accelerations, velocities, speeds, heights, and/or rest periods of the animal over time. The waste area 400 may include a memory, controller, and local user interface/display. The animal's movements may also, or alternatively, be monitored at the waste area using the sensors, devices (*e.g*., measuring devices), *etc.,* located on an animal (*e.g.,* an activity collar), as described herein.

Waste area 400 may have a measuring device, such as measuring device 420. As described herein, measuring device 420 may be one or more weighing scales, weight transducers, force transducers, displacement transducers, pressure transducers, weight sensors, force sensors, displacement sensors, pressure sensors, real time clocks, timers, counters, and/or a combination thereof. Measuring device 420 may include one or more photo electric sensors, such as a diffuse-reflective, through-beam, retro-reflective, and/or distance-settable sensor. For example, an area may be defined by a beam of light. When the beam of light is disrupted, it may be determined that the animal passed into the area or out of the area. Measurement device may include a thermometer and/or a microphone that may be used to determine the presence or absence of an animal in an area. For example, urine and/or feces deposited by an animal within an area (*e.g.,* a waste area) may change (*e.g.,* increase) the temperature of the area or the temperature of the animal. Microphones may be used to determine the presence of the animal or activities of the animal (such as urination, defecating, or urination of the animals).

Measuring device 420 may be used to measure the weight and/or pressure of an animal located at or near a waste area. Measuring device 420 may be used to measure one or more weights, pressures, *etc.,* at the waste area or around the waste area. For example, measuring device 420 may be used to measure the weight of an animal in the litter box, pressures incurred from the animal (*e.g.,* pressures induced from the paw of the animal), *etc.,* including a combination thereof. The measuring device 420 may be used to measure a pressure of the animal, for example, so that the measuring device can identify when an animal has entered, approached, passed by, *etc.,* the waste area. The measuring device 420 may be used to measure a pressure of the animal so that the gait of the animal may be determined. For example, a leg of an animal may exert more pressure on the ground as the acceleration, velocity, and/or speed of the animal increases.

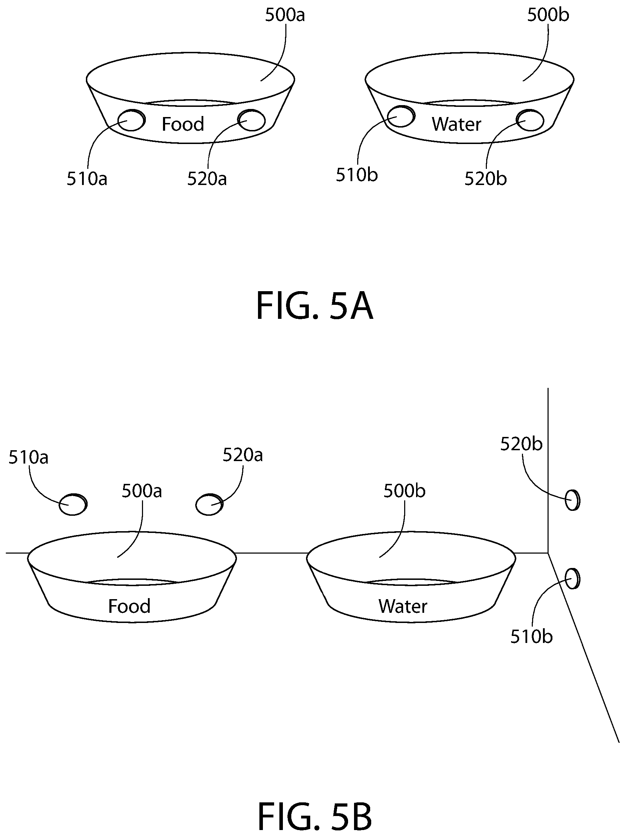

FIGS. 5A, 5B show example feeding and drinking areas that may be used to monitor an animal's movements, for example, to determine a gait of the animal (*e.g*., the gait of the animal at or near the feeding and drinking areas). The animal's gait may be monitored at the feeding and/or drinking areas using the sensors, devices (*e.g.,* measuring devices), *etc.,* located at the feeding and/or drinking area. For example, feeding bowl 500a and/or drinking bowl 500b may include communication circuitry, such as Bluetooth, RFID, Wi-Fi, and other wireless technologies. The feeding bowl 500a and/or drinking bowl 500b may communicate with an activity collar (such as mechanism 300) and/or a server. Feeding bowl 500a and/or drinking bowl 500b may communicate directly with a portable electronic device of the user, or such communication may occur indirectly via a server and an application, such as a web application.

The feeding bowl 500a and/or drinking bowl 500b may include a proximity sensor, such as a camera, scale, *etc.,* to track the gait of the animal at or proximate the feeding bowl 500a and/or drinking bowl 500b over time. The feeding bowl 500a and/or drinking bowl 500b may include a memory, controller, and local user interface/display. The animal's gait may also, or alternatively, be monitored at the feeding and/or drinking area using the sensors, devices (*e.g.,* measuring devices), *etc.,* located on an animal (*e.g.,* an activity collar), as described herein. Although the feeding and/or drinking area is shown as a feeding bowl 500 and a drinking bowl 500b, it should be understood that a feeding and/or drinking bowl may be different form factors than shown on FIGS 5A, 5B. For example, a drinking apparatus may include any device used by the animal to eat and/or drink. For example, the drinking apparatus may be a water bottle (*e.g.,* as used by a guinea pig, bunny), a sponge, an elevated pool, *etc.*

As shown on FIG. 5A, food dish 500a and/or a drinking bowl 500b may be an area designated for an animal (*e.g.,* a cat) to eat and/or drink. The food dish 500a and/or drinking bowl 500b may have one or more sensors. For example, a sensor may be located on the food dish and the water bowl, the food dish and not the water bowl, or *vice-versa.* The one or more sensors may be example sensors 510a, 510b, shown on FIGS. 5A and 5B. As shown on FIG. 5A, sensors 510a, 510b may be located on a portion of the eating and/or drinking area, such as on food dish 500a and/or a drinking bowl 500b. Sensors 510a, 510b may not be located on a portion of the eating and/or drinking area. For example, as shown on FIG. 5B, sensors 510a, 510b may be located on a wall, a table, *etc.,* or any other surface that may be in a predefined proximity to the eating and/or drinking area. Sensors 510a, 510b may be one or more motion sensors (such as an accelerometer, a gyroscope, a magnetometer, *etc*.), proximity sensors, orientation sensors, location sensors, and/or one or more other sensors, as described herein.

Food dish 500a and/or a drinking bowl 500b may have a measuring device, such as measuring devices 520a, 520b. As described herein, measuring devices 520a, 520b may be one or more weighing scales, weight transducers, force transducers, displacement transducers, pressure transducers, weight sensors, force sensors, displacement sensors, pressure sensors, real time clocks, timers, counters, and/or a combination thereof. Measuring device may be used to measure the weight and/or pressure of the animal at or near the eating and/or drinking area, in an example. Measuring device may be used to measure the weight and/or pressure of food and/or drink located at or near the eating and/or drinking area. Measuring devices 520a, 520b may be used to measure one or more weights, pressures, *etc.,* at the eating and/or drinking area or around the eating and/or drinking area.

As described herein, the gait of the animal during the first predetermined time period is determined based on movement data of the animal (which may be characterized by motion data and orientation data) during the first predetermined time period. Motion data may relate to whether and/or how one or more parts of the animal, such as one or more of the legs of the animal, are moving. Orientation data may relate to whether and/or how one or more parts of the animal, such as the animal's head, is pointed in an upward direction or a downward direction. The gait of the animal may be determined based on location data (*e.g.,* if the animal moves from one location to another location). The gait of the animal may be determined based on a combination of location data, orientation data, and/or motion data. For example, the gait of the animal may be determined based if the animal is moving at a high acceleration/velocity/speed with a head pointed in an upward direction to get from one location of the pet's yard to another location of the yard. The gait of the animal may include the walk, trot, canter, amble, pace, gallop, *etc.* of the animal.

The motion data of the animal may derive from one or more sensors, such as one or more accelerometers placed on a collar of the animal, the legs of the animal, the torso, of the animal, and the like. As an example, an accelerometer placed on the collar of the animal may determine (*e.g.,* sense) the movement of the dog. Sensor data (*e.g.,* accelerometer data) received from the collar of the animal may be associated with data associated with one or more appendages (*e.g*., legs, arms, neck) of the animal. For example, accelerometer data received from the collar of the animal may be associated with movement data related to the animal's legs. The movement data related to the animal's legs may be used to determine the gait of the animal.

As an example, sensor data may be received from a sensor (*e.g.,* an accelerometer) located and/or coupled to an article (*e.g.,* a collar) located on an animal. The sensor data may be normalized and/or transformed to account for movement of the collar around the animal, such as rotation of the collar around the neck of the animal. The sensor data may be normalized by determining the direction of the acceleration (*e.g.*, linear acceleration) component of the accelerometry signal and/or adjusting the values for the x, y, and z-axes.

As described herein, the accelerometer data derived from the collar of the animal may be used to determine movement data (*e.g*., acceleration, velocity, direction, location) of one or more appendages (*e.g*., legs) of the animal. By determining the movement data of the legs of the animal, for example, it may be determined whether the animal is walking, trotting, cantering, ambling, pacing, galloping, resting, *etc.* Further, by determining the times at which the appendages of the animal are moving, it may be determining how long and/or how often the animal is walking, trotting, cantering, ambling, pacing, galloping, resting, and/or has walked, trotted, cantered, ambled, paced, galloped, rested. *etc.* Such information may be used to determine how active (or inactive) the animal is, and/or whether an animal is injured, sick, immobile, healthy, *etc.* For example, an animal that is running for a predetermined amount of time may be considered a healthy animal. An animal that is resting for a predetermined amount of time may be considered an unhealthy animal, *etc.*

The sensor data associated with the animal may be associated with periods of times. The periods of times may be associated with frames. A sliding window may be used to divide the sensor data into frames containing short periods of data. For example, the frames may be in milliseconds (*e.g.,* 50 milliseconds), seconds (*e.g.,* 10 seconds), minutes (*e.g.,* 30 minutes), hours (*e.g.,* 2 hours), days, weeks, *etc.* The sensor data within the frames may be used to determine the gait of the animal within the frames. Sensor data within an overlap of the frames may be determined. The sensor data within the overlap of the frames may be used to account for the sensor data animal transitioning from one gait to another gait.

A range of features may be determined (*e.g*., calculated) relating to the sensor data. For example, the entropy, magnitude, kurtosis, signal energy, standard deviation, *etc.,* of the sensor data may be determined. Distributions of the entropy, magnitude, kurtosis, signal energy, standard deviation, *etc.,* of the sensor data may be analyzed to assess one or more values (*e.g.*, ranges of values) expected within each gait category. For example, the standard deviation of the sensor data of the animal may be determined. The determined standard deviation may be compared to predefined standard deviation associated with a gait of an animal. Based on the comparison of the determined standard deviation with the predefined standard deviation, the gait of the animal may be determined.

Machine learning techniques may be used to determine the gait of the animal, for example, based on the sensor data. Sensor data may include training data, test data, and/or validation data. For example, sensor data may be collected to produce a collection of training data. The training data may be sensor data examples used during the learning process. The training data may be used to fit sensor data, for example, to determine sensor data that may be associated with one or more gaits of the animal.

The training data may be used to train one or more networks (e.g., neural networks). The properties (*e.g*., specifications) of the networks and/or layers of the networks may determine portions of the network that may be activated based on incoming data (*e.g.,* incoming sensor data). The network may include one or more networks, such as one or more Long short-term memory (LSTM) networks. As known by those of ordinary skill in the art, the one or more LSTM networks may include an artificial recurrent neural network (RNN) architecture. The LSTM networks may include feedback connections. The LSTM networks may process positions, movements, and/or orientations of the animal. For example, the LSTM networks may process and/or provide inertial measurement unit (IMU) data provided via an electronic device, such as via an accelerometer, gyroscope, magnetometer, and the like. The LSTM unit may be composed of a cell, an input gate, an output gate, and/or a forget gate. The cell may recall values over time intervals (*e.g*., arbitrary time intervals) and/or one or more of the gates may regulate the flow of information into and out of the cell.

The network may include one or more (*e.g*., two) LSTM layers, a dropout layer, a dense rectified linear unit (ReLU) activation layer, and/or a dense softmax activation layer. The structure of the network (*e.g*., model) may be determined empirically. Hyperparameters may be optimized through one or more approaches, such as via a grid search approach. The network (*e.g*., model) may use categorical cross-entropy as a loss function and may employ an Adam optimizer. The network may be trained via an iterative process in which the network trains on the training set and is tested (*e.g.,* then tested) on the test set. The network may be adjusted according to the optimization algorithm, and the loss may be calculated. This process may be repeated for a set number of iterations, for a set period of time, and/or until the loss reaches a predetermined threshold.

Once the training process is completed the network may be provided validation data, which may have been previously unseen by the system. The validation data may be used to establish performance metrics. The validation data may exclude data that falls outside the expected feature ranges for one or more gaits of the animal. The ranges may be identified in the stage prior to the validation stage, such as the preprocessing stage. Excluding data may result in data (*e.g.,* only data) that is representative of the gait or falling within the same range, excluding a large portion of non-salient data.

In order to validate the performance of the data, the network may be tested on data collected in one or more contexts (*e.g*., one or more different contexts). Testing data in a different context may identify the generalizability of the data, such as the generalizability of the classifier. For example, during data collection a range of one or more (*e.g*., different) modes of forward motion may be identified. The classifier may be used to infer the gaits being exhibited at each time point in the data. Classification performance metrics may be (*e.g.,* may then be) calculated by assessing the relationship between the predicted labels and the annotations.

As described herein, one or more data sources may be used to determine the motion (*e.g*., forward motion, such as gait) of an animal. For example, one or more motion sensors (such as an accelerometer, a gyroscope, a magnetometer, *etc*.), proximity sensors, orientation sensors, location sensors, *etc.,* may be used to determine the motion (*e.g.,* gait) of an animal. The data sources may be placed on the animal (such as on a collar of the animal) and/or near an animal (such as on a feeding bowl, waste area, rest area, play area). Through the collection and/or combination of two or more data sources (*e.g*., data sources having complimentary sensing platforms) a framework for the assessment of animal motion (*e.g*., forward motion, such as gait) may be determined. For example, a pressure sensor located on a floor mat may provide information regarding the weight distribution of the animal while the animal is moving forward, backward, and/or sideways. Pressure sensor data combined with accelerometer data (*e.g*., accelerometer data recorded concurrently with the pressure sensor data) may identify patterns in the accelerometer data that may be representative of the data from the pressure sensor. Identifying patterns in accelerometer data that may be representative of pressure sensor data may provide an analysis of the mode of motion (*e.g*., forward motion, such as gait information) that may be inaccessible via accelerometer data alone.

Based on one or more of the animal's motion, location, orientation, *etc.,* a signature of the gait of the animal may be determined. The signature of the gait may include legs of the animal moving above a predefined acceleration and/or velocity if the animal is running, the height of the animal moving above a predetermined height if the animal is galloping or jumping, the orientation of the animal *(e.g.,* animal's legs) if the animal is resting, *etc.* Based on the movement of the animal being associated with a signature, the gait of the animal may be determined. As another example, the acceleration and/or velocity of an animal may be determined via one or more sensors (*e.g*., accelerometer) being located on an animal.

If the accelerometer is located on the animal (*e.g.*, the collar of the animal), the acceleration and/or velocity of the animal may be associated with the acceleration and/or velocity in which the neck of the animal is moving. The acceleration and/or velocity in which the neck of the animal is moving may be converted (*e.g*., transformed) to the acceleration and/or velocity in which one or more appendages (*e.g*., legs) of the animal is moving. If the legs of the animal are moving at an acceleration and/or velocity that is less than the acceleration and/or velocity at which an animal's legs would be moving if the animal were running (*e.g.,* a non-running signature), the animal may be determined to not be running. In such example, the animal may be determined to be walking, trotting, resting, *etc.* Alternatively, if the legs of the animal are moving at an acceleration and/or velocity that is greater than the acceleration and/or velocity at which an animal's legs would be moving if the animal were running (*e.g.,* a running signature), the animal may be determined to be running. In such example, the animal may be determined to be walking, trotting, resting, *etc.*

Mathematical and/or algorithmic techniques, such as bivariate, multivariate and trend analysis, may be used to formulate a trend of the movements of the animal (*e.g*., running, trotting, resting, *etc*.). Data collected over time and processed can represent a typical profile of behavior and habits of an animal. The behavior and habits of the animal may be used to determine the animal's gait. For example, an injured or otherwise ill animal may exhibit different movement habits than a healthy animal. Trend analysis may be used to determine whether the monitored behavior, habits, *etc.* of the animal are random, or whether a trend may be developing.

Data may be captured for the duration of the animal's activity and/or inactivity. Data may be captured by periodically sampling a sensor or sensors, such as a motion sensor (*e.g*., an accelerometer, gyroscope, or the like), a proximity sensor (*e.g.*, such as a camera or the like), *etc.* An array of digital data may be processed, for example, to extract a motion or non-motion of the animal (*e.g.,* walking, trotting, resting, *etc*.). The data may be processed inside a device (*e.g.,* a user device) on-the-fly (*e.g.,* applying methods as the data samples come in and not storing the entire data). Data may be stored in the device (in full length or a portion). Data may be processed with a delay, for example, in the device. Data may be processed externally from the device. For example, the data may be processed in a server, in a portable electronic device, and/or in a database that may perform the processing of the data.

Notifications may be delivered to the user, for example, in the form of an electronic mail message sent to a user-specified electronic mail address, a text message sent via SMS (Short Message Service) to a user-specified mobile phone number, a calendar reminder set up by the system in a user-specified calendar, phone calls to a user-specified mobile or landline phone number, messages by a mobile phone application of a user's mobile phone, *etc.*

According to the method of the invention, movement data of the animal for a predetermined time period are received via a sensor (102, 302, 310, 410, 510a, 510b), wherein the movement data comprises at least one of an acceleration of the animal during the first predetermined time period, a velocity of the animal during the first predetermined time period, a distance travelled by the animal during the first predetermined time period, a location of the animal during the first predetermined time period, or steps taken by the animal during the first predetermined time period. For example, the time and/or duration of a movement (*e.g*., a forward motion, rearward motion, sideward motion, side-to-side motion, *etc*.) of an animal may be recorded. For example, a date and/or time of the animal's running, jogging, jumping, resting, *etc.* may be recorded. Additionally, the time of year of the first predetermined time period and outdoor conditions at which the animal exercises the gait in the first predetermined time period are recorded. Orientations and/or locations of the animal may be recorded. All records may be stored and/or may be presented, for example, via a textual or graphical format.

A profile of the animal may be accessed via a portable electronic device. The portable electronic device may provide a user interface, for example, via an application downloaded on the portable electronic device. A user may create a profile associated with the animal. The application may display the animal's profile and/or may be facilitate the uploading of monitoring information of the animal, such as movements of the animal. Icons or symbols displayed on the application may designate one or more movements of the animal that may be monitored and/or tracked. For example, five bar icon may be shown to illustrate the animal running, three bar icons may be shown to illustrate the animal walking, zero bar icons may be shown to illustrate the animal sleeping, *etc.* Such data may be displayed in graph form for ease of reference.

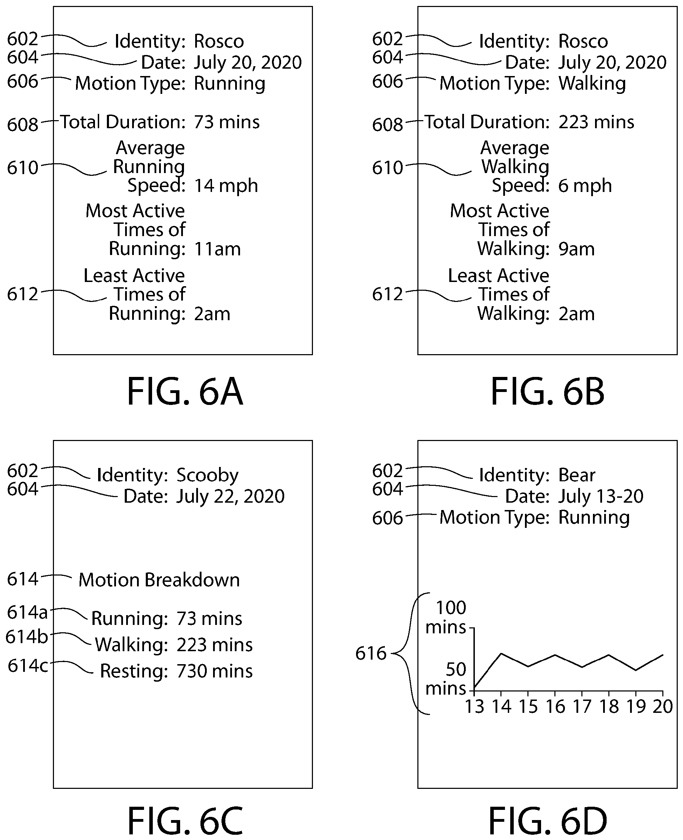

FIGS. 6A-6D show example screenshots of a use of the system determining movement information (*e.g.*, gait information) of an animal. The screenshots may be provided on a portable electronic device, for example. The screenshots provide information relating to the movements (*e.g*., motion type, duration of motion, average acceleration and/or velocity (or speed) of motion, motion direction, times at which the animal is most active in the motion, times in which the animal is least active in the motion, *etc.)* of the animal. The information shown on the screenshots are for illustration purposes only and are not limiting. In examples, other information (such as backwards motions, sideways motions, vertical motions, rest periods, *etc*.) may be provided to the user.

FIG. 6A shows an example screen shot of data collected and/or provided by one or more sensors, such as a mechanism (*e.g*., mechanism 200) worn by an animal, a proximity sensor located near the animal (*e.g.,* located at a feeding area, waste area, play area, *etc*.). Identity 602 shows the identity of the animal in which the motion (*e.g.,* gait) is being monitored, determined, and/or displayed. Although identity 602 shows the name of the animal on FIG. 6A, identity 602 may show one or more other types of information identifying the animal, such as the body type (*e.g., thin,* stocky, long, short) of the animal, the breed of the animal, a unique code identifying animal (such as a number), the pet owner's information, *etc.* The screenshots may be provided on a display, such as on a display of a portable electronic device.

According to the invention, movement data of the animal for a first predefined time period (such as Date 604) are received via a sensor. The time period defines the period of the data monitored and/or provided (*e.g.,* the period in which the motion of the animal, such as the animal's gait data, may be monitored and/or provided). Using the example shown on FIG. 6A, the gait data may be provided for the time period of a single day, such as July 20, 2020. In other examples, time period may be any time period, including multiple days, a week, a month, *etc.* Based on the desired time period, gait information (such as the motion type 606) may be provided. As shown on FIG. 6A, the motion type may a running of the animal, although in other examples other motion type information may be provided (such as the motion type 606 being walking), as shown on FIG. 6B.

Information relating the motion type 606 may be provided and/or determined. For example, as shown on FIGS. 6A, 6B, the duration 608 of the motion type 606, the average acceleration and/or velocity (or speed) 610 of the motion type 606, the most active times in which the motion type 606 occurs, and/or the least active times in which the motion type 606 occurs, may be provided and/or determined. The number of these events, and the listing of the events, is for illustration purposes only. Different (including more or less) categories of data, time periods, animal movements, *etc.,* may be displayed. For example, multiple motion types may be provided, averages and/or comparisons relating the different motion types may be provided, conditions relating to the motion type data may be provided (such as whether the animal is exhibiting a healthy condition or unhealthy condition based on the motion type data), recommendations based on the motion type may be provided (such as a recommendation to rest the animal and/or have the animal checked by a veterinarian) may be provided, *etc.*

As shown on FIG. 6C, screenshot may provide information (*e.g.*, motion breakdown 614) relating to one or more motion types. Motion breakdown 614 information may include one or more pieces of information relating to one or more motion types, such as the names of the motion types during a predetermined time period, the duration of one or more motion types, accelerations and/or velocities (or speeds) of the motion types, *etc.* For example, as shown on FIG. 6C, a user screenshot may show that for July 20, 2020, an animal may have run for 73 minutes, walked for 223 minutes, and/or rested for 730 minutes. As described herein, the numbers of these events, the listing of the events, *etc.,* is for illustration purposes only. Different (including more or less) categories of data may be displayed. For example, information relating to whether the animal is exercising, resting (*e.g*., sleeping) a sufficient amount or an insufficient amount may be determined. Condition information of the animal based on the movement may be provided and/or information relating to how to remedy a condition may be provided. For example, if an animal has run less than a predetermined amount during a time period, an indication may be provided for the caregiver of the animal to take the animal to the veterinarian.

As shown on FIG. 6D, information relating to the motion type of an animal may be graphically provided. For example, a screenshot may graphically show information relating to the running motion type 606 of an animal. The information shown graphically may relate to the duration in which the animal has run during the time period, the acceleration and/or velocity (or speed) at which the animal is moving during the time period, and the like. As shown on FIG. 6D, the graphical information relating the motion data may relate to the duration of the animal running during a seven day time period. Although FIG. 6D shows the time period being seven days and the graphical information showing information relating to the duration of the motion type, the numbers of these events, the listing of the events, *etc.,* is for illustration purposes only. Different (including more or less) categories of data may be displayed. More, or less, screen shots may be provided in which more or less data is presented to a user. The screenshots and/or data may be used for providing animal movement data *(e.g.,* forward motion data, such as gait data), animal signature data (*e.g.,* signature data of the gait), animal orientation data, animal location data, *etc.*

Based on the above, information relating to the gait of the animal may be provided. For example, a healthy animal may be expected to have certain gait characteristics (such as running above a predetermined amount of time), an unhealthy animal may be expected to have certain gait characteristics (such as running below a predetermined amount of time), an animal with a condition may be expected to have certain gait characteristics (such as an obese or aged animal running below a predetermined amount of time), *etc.*

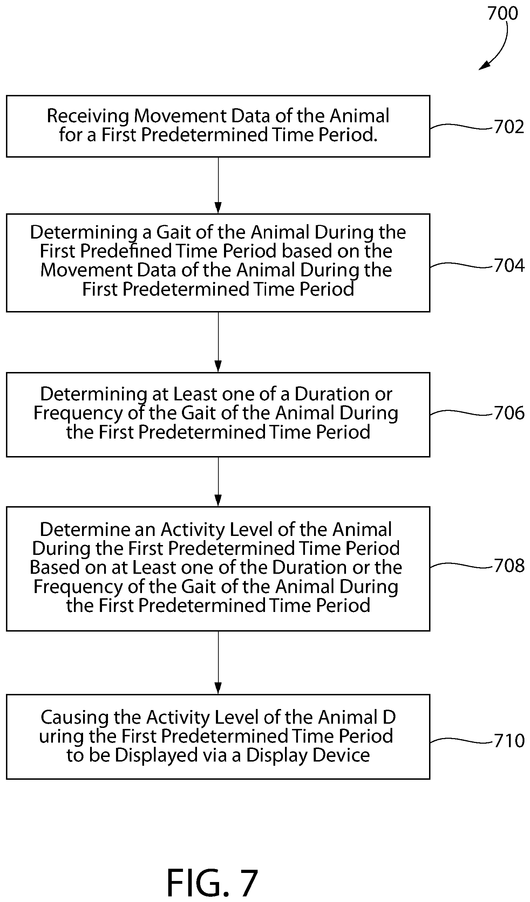

FIG. 7 describes a method 700 of monitoring of animal movement and/or motion data which may be useful in the method of the present invention if, at 702, movement data are received from a sensor (102, 302, 310, 410, 510a, 510b). The sensor may be one or more sensors, as described herein. For example, the sensor may be a sensor (or other device) configured to detect a location of the animal, to detect the motion (or stillness) of the animal, to detect an orientation of the animal, *etc.* The sensor may be one or more of a variety of form factors, as described herein. For the purposes of this disclosure, the sensor may include one or more measurement devices. As an example, the sensor may be one or more of an accelerometer, a gyroscope, a magnetometer, weighing scales, weight transducers, force transducers, displacement transducers, pressure transducers, weight sensors, force sensors, displacement sensors, pressure sensors, load cells, photographic cameras, video cameras, camcorders, contact thermometers, non-contact thermometers, and a combination thereof. Sensor may be one or more of optical sensors, optical reflecting sensors, LED/photodiode pair optical sensors, LED/phototransistor pair optical sensors, laser diode/photodiode pair optical sensors, laser diode/phototransistor pair optical sensors, optocouplers, optical fiber coupled optical sensors, magnetic sensors, weight sensors, force sensors, displacement sensors, pressure sensors, various proximity sensors, such as inductive proximity sensors, magnetic proximity sensors, capacitive proximity sensors, and/or a combination thereof. According to the invention, the movement data are associated with a first predetermined time period.

Movement data may be motion, location, orientation, *etc.,* data of an animal. The motion, location, orientation, *etc.,* data of the animal may be provided via one or more sensors or devices. The movement data may be associated with an hour, a day, a week, a month, *etc.* Movement data may be received from one or more other devices, such as a measuring device or one or more other sensors. Movement data may be received at a processor. The movement data may be associated with the movement of one or more portions of the animal's body. For example, if the sensor is located on collar of the animal on the neck of the animal, the sensor may detect movement of the neck of the animal. In other examples the sensor may be located on a leg, ear, tooth, torso, *etc.,* of the animal. In such examples the sensor may detect the movement of the respective area of the animal in which the sensor is located. As described herein, the movement of the animal may be determined in one or more locations in which the sensor is not located. For example, a sensor coupled to a collar may determine the movement of the neck of the animal. The movement of the neck of the animal may be used to determine the movement of one or more other locations of the animal, such as one or more appendages (*e.g*., legs) of the animal. According to the invention, the movement data comprises at least one of an acceleration of the animal during the first predetermined time period, a velocity of the animal during the first predetermined time period, a distance travelled by the animal during the first predetermined time period, a location of the animal during the first predetermined time period, or steps taken by the animal during the first predetermined time period.

At 704, a gait of the animal during the first predetermined time period is determined. The gait of the animal may include if and/or how the animal walks, trots, canters, ambles, gallops, *etc.* The gait of the animal may include the pace of the animal. According to the invention, the gait of the animal during the first predetermined time period is determined based on movement data of the animal during the first predetermined time period. The movement data may relate to one or more parts of the animal, such as movement of one or more of the appendages (*e.g.*, legs) of the animal and may be characterized by motion data, location data and orientation data. Orientation data may relate to one or more parts of the animal, such as the animal's head being pointed in an upward direction or a downward direction. The gait of the animal may be determined based on whether the animal moves from one location to another location. The gait of the animal may be determined based on a combination of location data, orientation data, and/or movement data. For example, the gait of the animal may be based on the animal moving in a forward direction at a high acceleration and/or velocity (or speed) with a head pointed in an upward direction travelling from one location to another location.

The gait of the animal may be allocated into one or more frames of data. Each of the frames may provide the gait of the animal during a period of time (*e.g.,* a short period of time). An overlap of the frames of the gait information may be provided. For example, one or more frames may provide an overlap of one gait (*e.g*., running) of the animal and another gait (*e.g*., walking) of the animal. The overlap may provide the transition of one gait of the animal and another gait of the animal. Data relating to features of the gait information may be determined. For example, the entropy, magnitude, kurtosis, signal energy, standard deviation, *etc.* of the data related to the gait of the animal may be determined.

At 706, the duration and/or frequency of the animal's gait is determined. For example, it may be determined how long and/or how many times the animal runs in a time period, walks in a time period, gallops in a time period, rests in a time period, and the like. As an example, it may be determined that an animal runs for ninety minutes in a twenty-four hour time period. The ninety minutes may include the animal running three times, for thirty minutes each time. As another example, it may be determined that an animal may rest for fifteen hours in a twenty-four hour period. The fifteen hours may include the animal sleeping for ten hours continuously at nighttime and resting for two additional one hundred and fifty minute periods.

At 708, the activity level of the animal is be determined for the predetermined time period. The activity level of the animal is based on the frequency and/or or duration of the gait of the animal during the predetermined time period. Using the example above, it may be determined that an animal has run for ninety minutes in a twenty-four hour time period. The running threshold for a healthy animal (*e.g*., healthy animal having a similar body type, breed, age, weight, sex, and/or medical condition of the animal) may be seventy five minutes in a twenty-four hour time period. By comparing the actual animal gait information against the threshold gait information, it may be determined that the animal's gait information is in line with a healthy animal.

Using the other example, it may be determined that the animal has rested for fifteen hours in a twenty-four hour period. The resting threshold for a healthy animal (*e.g*., healthy animal having a similar body type, breed, age, weight, sex, and/or medical condition of the animal) may be twelve hours in a twenty-four hour time period. By comparing the actual animal gait information against the threshold gait information, it may be determined that the animal's gait information is in line with an unhealthy animal. The care giver of the animal may be informed to seek medical attention for the animal based on whether it is determined that the animal is exhibiting a healthy gait condition or an unhealthy gait condition.

By comparing the actual animal gait information against the threshold gait information, it may be determined whether the animal has received the appropriate amount of exercise. For example, if an animal has not run for a predetermined amount of time during a time period, it may be determined that the animal has received an insufficient amount of exercise and/or an excess amount of rest that may cause health issues to the pet. The care giver may personally provide additional exercise to the pet, in some examples. In other examples, the animal may be boarded, and the pet parent can advise the boarder of the animal that the pet requires additional exercise.

Healthy and/or unhealthy threshold information may be updated based on machine learning techniques. For example, a machine learning model may initially be set to indicate that resting more than ten hours per twenty-four hour period is unhealthy. Based on training of the model with additional data sets, the threshold may be changed to indicate that resting ten to fourteen hours is indicative of a healthy animal and resting for more than fourteen hours per twenty-four hour period is unhealthy. The additional data sets may be updated based on veterinarian data. The additional data sets may be updated in real time, such as based on daily feedback provided by veterinarians in treating pets of various body types, breeds, weights, ages, sexes, medical conditions.

At 708, information relating to the animal, including the activity level of the animal for the predetermined time period is displayed. The activity level may include the gait of the animal, and a breakdown of the gait (such as the identity of the gait, the duration of the gait, the frequency of the gait.). For example, information indicating that the animal has ran for ninety minutes may be provided. As described herein, information associated with the animal's activity level is provided. For example, if the animal's activity exhibits a healthy condition of the animal, such information may be provided. Alternatively, if the animal's activity exhibits an unhealthy condition of the animal, such information may be provided. If the animal is determined to be exhibiting an unhealthy condition, remedial information may be provided. Remedial information may include information that the pet owner can perform (such as giving an over the counter medicine) and/or an indication that the animal should be seen by a veterinarian. Information relating to the activity level of the animal may be displayed on a display of a portable electronic device, such as a mobile phone, a tablet, or a mobile phone.

## Claims

1. A method for determining a health condition of an animal comprising:
receiving, via a sensor (102, 302, 310, 410, 510a, 510b), movement data of the animal for a first predetermined time period, wherein the movement data comprises at least one of an acceleration of the animal during the first predetermined time period, a velocity of the animal during the first predetermined time period, a distance travelled by the animal during the first predetermined time period, a location of the animal during the first predetermined time period, or steps taken by the animal during the first predetermined time period;
determining, via one or more processors (110), a gait of the animal during the first predetermined time period based on the movement data of the animal during the first predetermined time period;
determining at least one of a duration or frequency of the gait of the animal during the first predetermined time period;
determining a time at which the animal is most active in the gait in the first predetermined time period;
determining a time at which the animal is least active in the gait in the first predetermined time period;
recording the time of year of the first predetermined time period;
recording outdoor conditions at which the animal exercises the gait in the first predetermined time period;
determining an activity level of the animal during the first predetermined time period based on at least one of the duration or the frequency of the gait of the animal during the first predetermined time period;
causing the activity level of the animal during the first predetermined time period to be displayed via a display device;
receiving, via one or more of the processors (110), animal characteristic data comprising at least one of an age of the animal, a weight of the animal, a body type of the animal, a sex of the animal, a body fat index (BFI) of the animal, a body condition score (BCS) of the animal, or a muscle condition score (MCS) of the animal; and
determining, via the one or more processors (110), the health condition of the animal based on the animal characteristic data, the activity level, the gait of the animal during the first predetermined time period, the time of the year of the first predetermined time period, and the outdoor conditions at which the animal exercises the gait in the first predetermined time period.

2. The method of claim 1 wherein the gait of the animal comprises at least one the animal trotting, the animal cantering, the animal crawling, the animal ambling, the animal pacing, or the animal galloping.

3. The method of any of the previous claims further comprising determining, via the one or more processors (110), the activity level of the animal over a second predetermined time period, the second predetermined time period being greater than the first predetermined time period.

4. The method of any of the previous claims further comprising determining a level in which the animal was exercised during the first predetermined time period based on the gait of the animal during the first predetermined time period.

5. The method of any of the previous claims wherein the sensor (102, 302, 310, 410, 510a, 510b) is coupled to an article worn by the animal, or is implanted within the animal, and wherein the sensor (102, 302, 310, 410, 510a, 510b) comprises at least one of an accelerometer, a gyroscope, a magnetometer, or a global positioning system (GPS) device.

6. A system for determining an activity level of an animal comprising:
a sensor (102, 302, 310, 410, 510a, 510b) configured to receive movement data of the animal for a first predetermined time period, wherein the movement data comprises at least one of an acceleration of the animal during the first predetermined time period, a velocity of the animal during the first predetermined time period, a distance travelled by the animal during the first predetermined time period, a location of the animal during the first predetermined time period, or steps taken by the animal during the first predetermined time period; and
one or more processors (110) configured to:
determine a gait of the animal during the first predetermined time period based on the movement data of the animal during the first predetermined time period;
determine at least one of a duration or frequency of the gait of the animal during the first predetermined time period;
determine a time at which the animal is most active in the gait in the first predetermined time period;
determine a time at which the animal is least active in the gait in the first predetermined time period;
record the time of year of the first predetermined time period;
record outdoor conditions at which the animal exercises the gait in the first predetermined time period;
determine an activity level of the animal during the first predetermined time period based on at least one of the duration or the frequency of the gait of the animal during the first predetermined time period;
cause the activity level of the animal during the first predetermined time period to be displayed via a display device;
receive animal characteristic data comprising at least one of an age of the animal, a weight of the animal, a body type of the animal, a sex of the animal, a body fat index (BFI) of the animal, a body condition score (BCS) of the animal, or a muscle condition score (MCS) of the animal; and
determine a health condition of the animal based on the animal characteristic, the activity level, the gait of the animal during the first predetermined time period, the time of the year of the first predetermined time period, and the outdoor conditions at which the animal exercises the gait in the first predetermined time period.

7. The system of claim 6 wherein the gait of the animal comprises at least one of the animal walking, trotting, cantering, ambling, pacing, or galloping.

8. The system of any of claims 6-7 wherein the processor (110) is further configured to track the degree of activity over a second predetermined time period, the second predetermined time period being greater than the first predetermined time period.

9. The system of any of claims 6-8 wherein the processor (110) is further configured to determine a level in which the animal was exercised during the first predetermined time period based on the gait of the animal during the first predetermined time period.

10. The system of any of claims 6-9 wherein the sensor is coupled to an article worn by the animal.

11. The system of any of claims 6-10 wherein the sensor (102, 302, 310, 410, 510a, 510b) comprises at least one of an accelerometer, a gyroscope, a magnetometer, or a global positioning system (GPS) device.

## Patentansprüche

1. Verfahren zum Bestimmen des Gesundheitszustands eines Tieres, umfassend:
Empfangen von Bewegungsdaten des Tieres für einen ersten vorbestimmten Zeitraum über einen Sensor (102, 302, 310, 410, 510a, 510b), wobei die Bewegungsdaten mindestens eines der folgenden Elemente umfassen: eine Beschleunigung des Tieres während des ersten vorbestimmten Zeitraums, eine Geschwindigkeit des Tieres während des ersten vorbestimmten Zeitraums, eine vom Tier während des ersten vorbestimmten Zeitraums zurückgelegte Strecke, einen Standort des Tieres während des ersten vorbestimmten Zeitraums oder die Schritte des Tieres während des ersten vorbestimmten Zeitraums;
Bestimmen einer Gangart des Tieres während des ersten vorbestimmten Zeitraums auf der Grundlage der Bewegungsdaten des Tieres während des ersten vorbestimmten Zeitraums über einen oder mehrere Prozessoren (110);
Bestimmen mindestens einer Dauer oder Häufigkeit der Gangart des Tieres während des ersten vorbestimmten Zeitraums;
Bestimmen eines Zeitpunkts, zu dem das Tier im ersten vorbestimmten Zeitraum in der Gangart am aktivsten ist;
Bestimmen eines Zeitpunkts, zu dem das Tier im ersten vorbestimmten Zeitraum in der Gangart am wenigsten aktiv ist;
Aufzeichnen der Jahreszeit des ersten vorbestimmten Zeitraums;
Aufzeichnen der Außenbedingungen, unter denen das Tier im ersten vorbestimmten Zeitraum die Gangart ausübt;
Bestimmen eines Aktivitätsniveaus des Tieres während des ersten vorbestimmten Zeitraums auf der Grundlage mindestens einer der Dauer oder der Häufigkeit der Gangart des Tieres während des ersten vorbestimmten Zeitraums;
Anzeigen des Aktivitätsniveaus des Tieres während des ersten vorbestimmten Zeitraums über eine Anzeigevorrichtung;
Empfangen von Tiercharakteristikdaten über einen oder mehrere der Prozessoren (110), die mindestens eines der folgenden Merkmale umfassen: Alter des Tieres, Gewicht des Tieres, Körpertyp des Tieres, Geschlecht des Tieres, Körperfettindex (BFI) des Tieres, Körperkonditionswert (BCS) des Tieres oder Muskelkonditionswert (MCS) des Tieres umfassen; und
Bestimmen des Gesundheitszustands des Tieres über den einen oder die mehreren Prozessoren (110) auf der Grundlage der Tiercharakteristikdaten, des Aktivitätsniveaus, der Gangart des Tieres während des ersten vorbestimmten Zeitraums, der Jahreszeit des ersten vorbestimmten Zeitraums und der Außenbedingungen, unter denen das Tier im ersten vorbestimmten Zeitraum die Gangart ausübt.

2. Verfahren nach Anspruch 1, wobei die Gangart des Tieres mindestens eine der folgenden Gangarten umfasst: Traben, Galoppieren, Kriechen, Schlendern, Schreiten oder Galoppieren.

3. Verfahren nach einem der vorhergehenden Ansprüche, das ferner das Bestimmen des Aktivitätsniveaus des Tieres über einen zweiten vorbestimmten Zeitraum mittels des einen oder der mehreren Prozessoren (110) umfasst, wobei der zweite vorbestimmte Zeitraum größer ist als der erste vorbestimmte Zeitraum.

4. Verfahren nach einem der vorhergehenden Ansprüche, das ferner das Bestimmen eines Niveaus, auf dem das Tier während des ersten vorbestimmten Zeitraums trainiert wurde, auf der Grundlage der Gangart des Tieres während des ersten vorbestimmten Zeitraums umfasst.

5. Verfahren nach einem der vorhergehenden Ansprüche, wobei der Sensor (102, 302, 310, 410, 510a, 510b) mit einem vom Tier getragenen Gegenstand verbunden oder in das Tier implantiert ist und wobei der Sensor (102, 302, 310, 410, 510a, 510b) mindestens eines von einem Beschleunigungsmesser, einem Gyroskop, einem Magnetometer oder einem Global-Positioning-System-(GPS-)Gerät umfasst.

6. System zum Bestimmen eines Aktivitätsniveaus eines Tieres, umfassend:
einen Sensor (102, 302, 310, 410, 510a, 510b), der so konfiguriert ist, dass er Bewegungsdaten des Tieres für einen ersten vorbestimmten Zeitraum empfängt, wobei die Bewegungsdaten mindestens eines der folgenden Elemente umfassen: eine Beschleunigung des Tieres während des ersten vorbestimmten Zeitraums, eine Geschwindigkeit des Tieres während des ersten vorbestimmten Zeitraums, eine vom Tier während des ersten vorbestimmten Zeitraums zurückgelegte Strecke, einen Standort des Tieres während des ersten vorbestimmten Zeitraums oder Schritte des Tieres während des ersten vorbestimmten Zeitraums; und
einen oder mehrere Prozessoren (110), die so konfiguriert sind, dass sie:
eine Gangart des Tieres während des ersten vorbestimmten Zeitraums auf der Grundlage der Bewegungsdaten des Tieres während des ersten vorbestimmten Zeitraums bestimmen;
mindestens eine der Dauer oder Häufigkeit der Gangart des Tieres während des ersten vorbestimmten Zeitraums bestimmen;
einen Zeitpunkt bestimmen, zu dem das Tier im ersten vorbestimmten Zeitraum in der Gangart am aktivsten ist;
einen Zeitpunkt zu bestimmen, zu dem das Tier im ersten vorbestimmten Zeitraum in der Gangart am wenigsten aktiv ist;
die Jahreszeit des ersten vorbestimmten Zeitraums aufzuzeichnen;
die Außenbedingungen aufzuzeichnen, unter denen das Tier im ersten vorbestimmten Zeitraum die Gangart ausübt;
ein Aktivitätsniveau des Tieres während des ersten vorbestimmten Zeitraums auf der Grundlage mindestens einer der Dauer oder der Häufigkeit der Gangart des Tieres während des ersten vorbestimmten Zeitraums zu bestimmen;
das Aktivitätsniveau des Tieres während des ersten vorbestimmten Zeitraums über eine Anzeigevorrichtung anzuzeigen;
Tiercharakteristikdaten, die mindestens eines der folgenden Merkmale umfassen: Alter des Tieres, Gewicht des Tieres, Körperbau des Tieres, Geschlecht des Tieres, Körperfettindex (BFI) des Tieres, Körperkonditionswert (BCS) des Tieres oder Muskelkonditionswert (MCS) des Tieres umfassen, zu bestimmen; und
Bestimmen eines Gesundheitszustands des Tieres auf der Grundlage der Tiercharakteristik, des Aktivitätsniveaus, der Gangart des Tieres während des ersten vorbestimmten Zeitraums, der Jahreszeit des ersten vorbestimmten Zeitraums und der Außenbedingungen, unter denen das Tier im ersten vorbestimmten Zeitraum die Gangart ausübt.

7. System nach Anspruch 6, wobei die Gangart des Tieres mindestens eines der folgenden Gangarten umfasst: Gehen, Traben, Galoppieren, Schlendern, Schreiten oder Galoppieren.

8. System nach einem der Ansprüche 6-7, wobei der Prozessor (110) ferner so konfiguriert ist, dass er den Aktivitätsgrad über einen zweiten vorbestimmten Zeitraum verfolgt, wobei der zweite vorbestimmte Zeitraum länger ist als der erste vorbestimmte Zeitraum.

9. System nach einem der Ansprüche 6-8, wobei der Prozessor (110) ferner so konfiguriert ist, dass er auf der Grundlage der Gangart des Tieres während des ersten vorbestimmten Zeitraums ein Niveau bestimmt, auf dem das Tier während des ersten vorbestimmten Zeitraums trainiert wurde.

10. System nach einem der Ansprüche 6-9, wobei der Sensor mit einem vom Tier getragenen Gegenstand gekoppelt ist.

11. System nach einem der Ansprüche 6-10, wobei der Sensor (102, 302, 310, 410, 510a, 510b) mindestens eines von einem Beschleunigungsmesser, einem Gyroskop, einem Magnetometer oder einer Global-Positioning-System-(GPS-)Gerät umfasst.

## Revendications

1. Procédé de détermination de l'état de santé d'un animal, comprenant :
la réception, via un capteur (102, 302, 310, 410, 510a, 510b), de données de mouvement de l'animal sur une première période de temps prédéterminée, dans laquelle les données de mouvement comprennent au moins un élément parmi une accélération de l'animal pendant la première période de temps prédéterminée, une vitesse de l'animal pendant la première période de temps prédéterminée, une distance parcourue par l'animal pendant la première période de temps prédéterminée, une position de l'animal pendant la première période de temps prédéterminée ou le nombre de pas de l'animal pendant la première période de temps prédéterminée ;
la détermination, par un ou plusieurs processeurs (110), de la démarche de l'animal pendant la première période de temps prédéterminée sur la base des données de mouvement de l'animal pendant la première période de temps prédéterminée ;
la détermination d'au moins un élément parmi la durée ou la fréquence de la démarche de l'animal pendant la première période de temps prédéterminée ;
la détermination d'un moment où l'animal est le plus actif dans la démarche pendant la première période de temps prédéterminée ;
la détermination du moment où l'animal est le moins actif dans la démarche pendant la première période de temps prédéterminée ;
l'enregistrement de la période de l'année de la première période de temps prédéterminée ;
l'enregistrement des conditions extérieures dans lesquelles l'animal pratique la démarche dans la première période de temps prédéterminée ;
la détermination d'un niveau d'activité de l'animal pendant la première période de temps prédéterminée, sur la base d'au moins un élément parmi la durée ou la fréquence de la démarche de l'animal pendant la première période de temps prédéterminée ;
l'affichage du niveau d'activité de l'animal pendant la première période de temps prédéterminée via un dispositif d'affichage ;
la réception, par un ou plusieurs des processeurs (110), de données caractéristiques de l'animal comprenant au moins un élément parmi l'âge de l'animal, le poids de l'animal, la morphologie de l'animal, le sexe de l'animal, l'indice de masse grasse (BFI) de l'animal, le score de l'état corporel (BCS) de l'animal ou le score de l'état musculaire (MCS) de l'animal ; et
la détermination, par les un ou plusieurs processeurs (110), de l'état de santé de l'animal sur la base des données caractéristiques de l'animal, du niveau d'activité, de la démarche de l'animal pendant la première période de temps prédéterminée, de la période de l'année de la première période de temps prédéterminée et les conditions extérieures dans lesquelles l'animal pratique la démarche dans la première période de temps prédéterminée.

2. Procédé selon la revendication 1, dans lequel la démarche de l'animal comprend au moins une démarche parmi : l'animal au trot, l'animal au petit galop, l'animal qui rampe, l'animal à l'amble, l'animal au pas trotté, ou l'animal au galop rapide.

3. Procédé selon l'une des revendications précédentes, comprenant en outre la détermination, par les un ou plusieurs processeurs (110), du niveau d'activité de l'animal sur une seconde période de temps prédéterminée, la seconde période de temps prédéterminée étant supérieure à la première période de temps prédéterminée.

4. Procédé selon l'une des revendications précédentes, comprenant en outre la détermination d'un niveau d'activité physique auquel l'animal a été soumis pendant la première période de temps prédéterminée, sur la base de la démarche de l'animal pendant la première période de temps prédéterminée.

5. Procédé selon l'une des revendications précédentes, dans lequel le capteur (102, 302, 310, 410, 510a, 510b) est accouplé à un accessoire porté par l'animal ou implanté dans l'animal, et dans lequel le capteur (102, 302, 310, 410, 510a, 510b) comprend au moins un élément parmi un accéléromètre, un gyroscope, un magnétomètre ou un dispositif à système de positionnement mondial (GPS).

6. Système de détermination d'un niveau d'activité d'un animal, comprenant :
un capteur (102, 302, 310, 410, 510a, 510b) configuré pour recevoir des données de mouvement de l'animal pendant une première période de temps prédéterminée, dans lequel les données de mouvement comprennent au moins un élément parmi une accélération de l'animal pendant la première période de temps prédéterminée, une vitesse de l'animal pendant la première période de temps prédéterminée, une distance parcourue par l'animal pendant la première période de temps prédéterminée, une position de l'animal pendant la première période de temps prédéterminée ou le nombre de pas de l'animal pendant la première période de temps prédéterminée ; et
un ou plusieurs processeurs (110) configurés pour :
déterminer une démarche de l'animal pendant la première période de temps prédéterminée sur la base des données de mouvement de l'animal pendant la première période de temps prédéterminée ;
déterminer au moins un élément parmi la durée ou la fréquence de la démarche de l'animal pendant la première période de temps prédéterminée ;
déterminer le moment où l'animal est le plus actif dans la démarche pendant la première période de temps prédéterminée ;
déterminer le moment où l'animal est le moins actif dans la démarche pendant la première période de temps prédéterminée ;
enregistrer la période de l'année correspondant à la première période de temps prédéterminée ;
enregistrer les conditions extérieures dans lesquelles l'animal pratique la démarche dans la première période de temps prédéterminée ;
déterminer un niveau d'activité de l'animal pendant la première période de temps prédéterminée, sur la base d'au moins un élément parmi la durée ou la fréquence de la démarche de l'animal pendant la première période de temps prédéterminée ;
afficher le niveau d'activité de l'animal pendant la première période de temps prédéterminée via un dispositif d'affichage ;
recevoir des données caractéristiques de l'animal, comprenant au moins un élément parmi l'âge de l'animal, le poids de l'animal, la morphologie de l'animal, le sexe de l'animal, l'indice de masse grasse (BFI) de l'animal, le score de l'état corporel (BCS) de l'animal, le score de l'état musculaire (MCS) de l'animal ; et
déterminer un état de santé de l'animal sur la base des caractéristiques de l'animal, du niveau d'activité, de la démarche de l'animal pendant la première période de temps prédéterminée, de la période de l'année correspondant à la première période de temps prédéterminée et des conditions extérieures dans lesquelles l'animal pratique la démarche dans la première période de temps prédéterminée.

7. Système selon la revendication 6, dans lequel la démarche de l'animal comprend au moins une démarche parmi : l'animal qui marche, au trot, au petit galop, à l'amble, au pas trotté ou au galop rapide.

8. Système selon l'une des revendications 6 à 7, dans lequel le processeur (110) est en outre configuré pour suivre le degré d'activité sur une seconde période de temps prédéterminée, la seconde période de temps prédéterminée étant supérieure à la première période de temps prédéterminée.

9. Système selon l'une des revendications 6 à 8, dans lequel le processeur (110) est en outre configuré pour déterminer un niveau d'activité physique auquel l'animal a été soumis pendant la première période de temps prédéterminée, sur la base de la démarche de l'animal pendant la première période de temps prédéterminée.

10. Système selon l'une des revendications 6 à 9, dans lequel le capteur est accouplé à un accessoire porté par l'animal.

11. Système selon l'une des revendications 6 à 10, dans lequel le capteur (102, 302, 310, 410, 510a, 510b) comprend au moins un élément parmi un accéléromètre, un gyroscope, un magnétomètre ou un dispositif à système de positionnement mondial (GPS).
